# FASCICULE DE BREVET EUROPEEN

(11) **EP 2 619 625 B1**
(45) Date de publication et mention de la délivrance du brevet: **14.11.2018**
(21) Numéro de dépôt: 11773758.5
(22) Date de dépôt: 23.09.2011
(51) Int. Cl.: A61B 3/113, G02C 7/06, A61B 3/14, G02C 13/00

(54) **METHODE DE SELECTION DE LENTILLES OPHTALMIQUES PROGRESSIVES**
VERFAHREN ZUR AUSWAHL VON GLEITSICHTGLÄSERN
METHOD FOR SELECTING PROGRESSIVE OPHTHALMIC LENSES

(30) Priorité: 23.09.2010 FR 1057684
(43) Date de publication de la demande: 31.07.2013
(73) Titulaire: ACEP France, 75017 Paris (FR)
(72) Inventeur: SAYAG, Jean-Philippe, 75017 Paris (FR); GRILLON, Benoit, 75017 Paris (FR)
(74) Mandataire: Oudin, Stéphane
(86) Numéro de dépôt international: PCT/FR2011/052215
(87) Numéro de publication internationale: WO 2012/038676

(56) Documents cités:
- EP-A2- 1 038 495
- FR-A1- 2 896 682
- FR-A1- 2 931 002

## Description

La présente invention se rapporte à une méthode de sélection de lentilles ophtalmiques progressives pour une monture donnée chaussée par un porteur déterminé.

Les lentilles ophtalmiques progressives sont associées à une prescription médicale et elles sont enchâssées dans une monture que le porteur choisi en fonction de ses goûts.

Ce type de lentilles présente une zone de vision de loin, située dans la partie supérieure de la lentille et une zone de vision de près située dans la partie inférieure. Ces zones de vision présentent des caractéristiques optiques différentes. Aussi, et c'est là une particularité de ce type de lentille ophtalmique, elle présente un couloir de progression qui s'étend d'une zone à l'autre, et dont les caractéristiques optiques varient de manière continue. Ainsi, de la zone de vision de loin vers la zone de vision de près, les caractéristiques optiques de la zone de vision de loin évoluent progressivement vers les caractéristiques optiques de la zone de vision de près. Cela permet au porteur de passer d'une zone à l'autre sans désagrément et avec un certain confort.

Cependant, ce couloir de progression et notamment sa longueur peut varier d'une monture à l'autre. On comprend en effet que la longueur de progression varie en fonction de la position des lentilles ophtalmiques par rapport à l'oeil, et par conséquent par rapport à la monture choisie.

Aussi, il a été imaginé de prendre en compte la taille et la forme de la monture à des fins d'optimiser la lentille ophtalmique progressive et apporter un confort optimale au porteur. On pourra notamment se référer au document FR 2 898 193, lequel décrit un procédé de détermination d'une lentille ophtalmique progressive adaptée au porteur et à la monture choisie.

Le procédé fait appel à des paramètres représentatifs obtenus à partir de valeurs moyennes elles-mêmes calculées à partir d'un échantillon de population donnée. Malgré ces précautions, il s'avère que certaines personnes éprouvent des difficultés à supporter leurs lentilles ophtalmiques progressives.

Aussi, un problème qui se pose et que vise à résoudre la présente invention est de fournir une méthode de sélection de lentilles ophtalmiques progressives en fonction des caractéristiques du porteur et de la monture qu'il choisit, de manière à accroître plus encore son confort de vision.

Dans ce but, la présente invention propose une méthode de sélection de lentilles ophtalmiques progressives pour une monture et un porteur donnés, les lentilles ophtalmiques progressives présentant une zone de vision de loin et une zone de vision de près espacées l'une de l'autre d'une longueur de progression, ladite monture donnée présentant deux logements aptes à recevoir respectivement une lentille ophtalmique progressive, lesdits deux logements définissant un plan moyen de logement. La demande de brevet Européenne EP 1 038 495 A2 décrit une méthode de sélection de lentilles ophtalmiques. Selon l'invention la méthode comprend les étapes récitées dans la revendication 1.

Ainsi, une caractéristique de l'invention réside dans la mesure personnalisée des positions relatives des points d'intersection du plan moyen de logement, respectivement avec les directions du regard du porteur en vision de loin et en vision de près et dans l'évaluation de la distance qui sépare ces points d'intersection pour sélectionner des lentilles ophtalmiques progressives correspondantes. En pratique, on peut par exemple repérer respectivement ces points d'intersection par rapport au bord inférieur de la monture et mesurer verticalement la distance qui les en sépare. Il s'avère que la distance qui sépare ces points d'intersection diffère d'un patient à l'autre et d'une monture à l'autre et ce dans des proportions relativement importantes. Or, les lentilles ophtalmiques progressives sont proposées avec des longueurs de progression standards qui ne sont pas nécessairement adaptées en toutes circonstances pour un porteur donné chaussant une monture donnée. Grâce à la méthode selon invention, il est possible de choisir pour un porteur donné ayant choisi une monture les lentilles ophtalmiques progressives dont la longueur de progression est identique à la distance qui sépare les points d'intersection précitée. Au surplus, si toutefois aucunes lentilles ophtalmiques progressives ne correspondaient à cette distance, le choix de la monture pourrait être remis en cause de manière à identifier une monture convenable.

La direction du regard du porteur dans une posture de vision de loin correspond à la direction du regard lorsque qu'il est debout et qu'il observe l'horizon. Dans une posture de vision de près, la direction du regard est celle du porteur lorsqu'il lit dans une position naturelle, par exemple un livre qu'il tient dans ses mains.

Selon un mode de mise en oeuvre de l'invention particulièrement avantageux, on équipe ladite monture d'un équipement calibré présentant trois points de repères espacés respectivement les uns des autres d'une distance déterminée. Ainsi qu'on l'expliquera ci-après plus en détail, l'équipement calibré permet de mesurer la distance de certaines caractéristiques de la monture en relation avec certaines autres caractéristiques anthropométriques du porteur. On expliquera également le calcul de certaines de ces distances par triangulation. On observera que l'équipement calibré permet de s'affranchir de la mise en place de repères sur la monture elle-même. Toutefois, il est également possible d'utiliser l'équipement calibré pour mesurer certaine de ces distances est également la valeur de la hauteur de monture par exemple pour mesurer certaines autres distances. Préférentiellement, l'équipement calibré s'ajuste dans la partie supérieure de la monture et il présente deux points de repères latéraux voisins des branches de la monture et reliés par un pont, ainsi qu'un point de repère central situé à l'extrémité d'une tige montée perpendiculairement au pont entre les deux points de repères latéraux.

Selon une caractéristique particulièrement avantageuse, à l'étape b) précitées, on enregistre selon une direction voisine de ladite première direction du regard, une première image de ladite monture équipée dudit équipement calibré et des pupilles des yeux dudit porteur pour déterminer la position dudit premier point d'intersection par rapport à ladite monture. Ainsi qu'on l'expliquera ci-après, grâce à la première image et selon une variante de mise en oeuvre, on mesure la distance qui sépare le bord inférieur de la monture et le reflet cornéen du porteur ainsi que les distances relatives des trois points de repères précités pour déterminer l'angle du plan moyen de logement par rapport à un plan vertical. L'angle du plan moyen par rapport à la verticale est appelé angle pantoscopique. Grâce à ces éléments, on détermine la position du premier point d'intersection entre le plan moyen de logement et les lentilles ophtalmiques progressives, par rapport au bord inférieur de la monture.

Avantageusement, on fournit une première caméra et en ce on ajuste ladite première caméra à distance dudit porteur chaussant ladite monture équipée dudit équipement calibré et selon une direction voisine de ladite première direction du regard pour enregistrer ladite première image. La première caméra est par exemple ajustée à environ 2 m du porteur en position debout et à hauteur de ses yeux. De la sorte, l'image obtenue par la première caméra peut être traitée et analyser, et ce de manière automatique, ou bien semi-automatique, de manière à obtenir les valeurs précitées.

En outre, à l'étape c), on enregistre selon une direction voisine de ladite seconde direction du regard, une seconde image de ladite monture équipée dudit équipement calibré et des pupilles des yeux dudit porteur pour déterminer la position dudit second point d'intersection par rapport à ladite monture. Des mesures et un calcul comparable à ce de l'étape b) précitée peuvent être mises en oeuvre pour déterminer la position du second point d'intersection, par exemple par rapport au bord inférieur de la monture.

Aussi, pour ce faire, on fournit un support mobile portant des inscriptions et une seconde caméra solidaire dudit support mobile et installée à travers lesdites inscriptions, et on autorise ledit porteur chaussant ladite monture équipée dudit équipement calibré à ajuster ledit support mobile à une distance naturelle de lecture, pour enregistrer ladite seconde image. Ainsi, grâce à la seconde caméra embarquée sur le support portant les inscriptions, on pourra obtenir une image de la monture équipée de l'équipement et de la pupille des yeux du porteur depuis le support et selon une direction voisine de la seconde direction du regard du porteur. De la même manière, que pour la première caméra, l'image obtenue peut être traitée et analyser pour obtenir la position du second point d'intersection par rapport au bord inférieur de la monture.

On observera que ces mesures et ses calculs sont simplifiés lorsque l'axe optique de la caméra est confondu avec la direction du regard du porteur. Si on peut considérer que cela est le cas de la première direction du regard dans la première posture en vision de loin, il n'en est pas de même dans le cas de la seconde direction du regard dans la posture en vision de près.

Selon un autre objet, la présente invention concerne un ensemble de traitement automatique comprenant les caractéristiques de l'ensemble défini dans la revendication 6.

En outre, lesdits premiers et seconds moyens de détermination comprennent avantageusement un équipement calibré destiné à équiper ladite monture, et présentant trois points de repères espacés respectivement les uns des autres d'une distance déterminée. Cet équipement calibré permet notamment, comme on l'expliquera plus en détail ci-après, de déterminer par triangulation l'orientation des montures mais aussi, les distances au niveau des yeux du porteur.

De plus, et selon un mode de mise en oeuvre de l'invention particulièrement avantageux, lesdits seconds moyens de détermination comprennent un écran d'affichage portable et une caméra installée sur ledit écran d'affichage portable. L'écran d'affichage portable par exemple une « tablette PC », surmontée d'une caméra embarquée. Ainsi, et comme on l'expliquera ci-après plus en détail, le porteur peut lui-même ajuster l'écran d'affichage à la distance naturelle de vision de près et aussi, orienter l'axe optique de la caméra.

En outre, lesdits seconds moyens de détermination comprennent avantageusement des moyens d'enregistrement et de calcul, pour enregistrer les images fournies par ladite caméra et pour calculer ladite position du second point d'intersection de la seconde direction du regard dudit porteur dans une posture de vision de près avec ledit plan moyen de logement.

Des modes de réalisation additionnels sont définis dans les revendications dépendantes.

D'autres particularités et avantages de l'invention ressortiront à la lecture de la description faite ci-après d'un mode de réalisation particulier de l'invention, donné à titre indicatif mais non limitatif, en référence aux dessins annexés sur lesquels :
- la Figure 1 est une vue schématique de face des yeux d'un porteur de monture équipée d'un équipement calibré ;
- la Figure 2 est une vue schématique de côté montrant le porteur de monture équipée dans une posture de vision de loin;
- la Figure 3 est une vue schématique de détail de la Figure 2 ;
- la Figure 4 est une vue schématique de côté montrant le porteur de monture équipée dans une posture de vision de près;
- la Figure 5 est une vue schématique de détail de la Figure 4, correspondant à un exemple de méthode de sélection de lentilles ophtalmiques non revendiqué ; et,
- la figure 6 est une vue schématique de détail selon le mode principal de mise en oeuvre de l'invention.

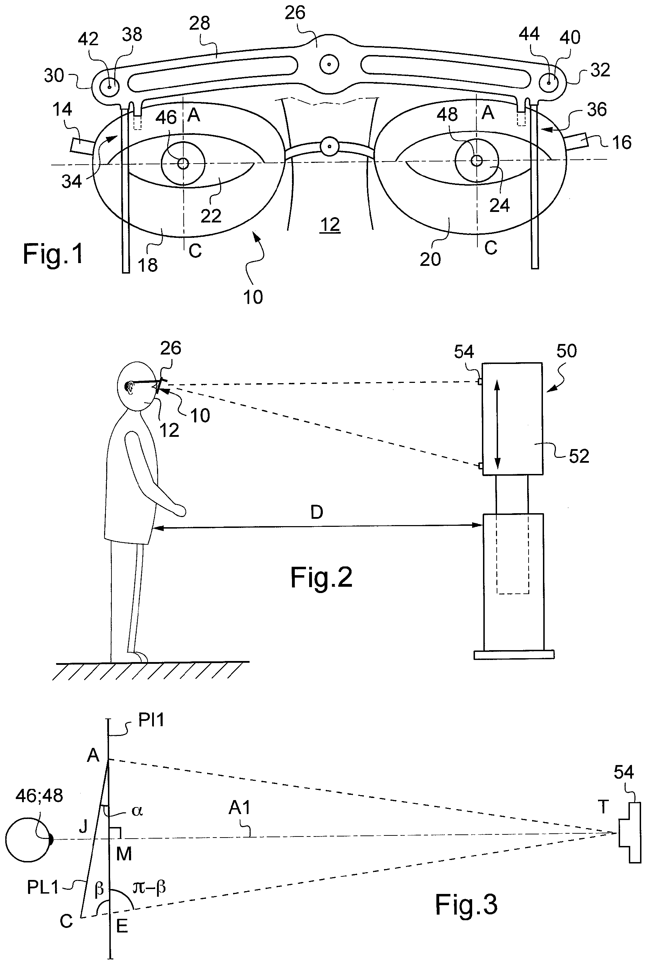

La Figure 1 illustre une monture 10 installée sur le visage 12 d'un porteur. Cette monture 10 présente deux branches 14, 16 et de deux logements 18, 20 délimités par une structure fermée sensiblement ovoïde et qui s'étendent respectivement devant les yeux 22, 24 du porteur pour recevoir des lentilles ophtalmiques progressives. En outre, la monture 10 est équipée d'un équipement calibré 26. Cet équipement calibré 26 présente un pont 28 qui s'étend longitudinalement et deux extrémités opposées 30, 32 lesquelles comportent respectivement une fourchette de fixation 34, 36. Ainsi, les fourchettes de fixation 34, 36 chevauchent la monture 10 au voisinage des deux branches respectives 14, 16 de cette dernière, en laissant libre l'espace visuel du porteur. L'équipement calibré 26 qui est ainsi totalement solidaire de la monture 10, présente deux repères opposés 38, 40 installés respectivement aux deux extrémités opposées 30, 32. Ces deux repères opposés 38, 40 présentent respectivement une marque de couleur blanche sous la forme d'un disque et au centre duquel est porté un point noir 42, 44. En outre, les deux points noirs 42, 44 sont espacés d'une distance connue, par exemple égale à exactement 110 mm.

Par ailleurs, on observera sur cette Figure les pupilles 46, 48 des yeux 22, 24 du porteur, lesquelles sont aptes à présenter en leur centre une marque blanche correspondant au reflet du sommet de la cornée. Ainsi, il apparaît en vue de face à la fois l'équipement calibré 26 et les deux points noirs 42, 44 espacés d'une distance déterminée et dans un plan vertical voisin extrêmement proche, la pupille 46, 48 des yeux 22, 24 du porteur.

En outre, la monture 10 présente une hauteur AC correspondant à la hauteur maximale de la structure fermée selon un plan sensiblement vertical coupant les pupilles 46, 48. Les points A et C correspondent respectivement aux bords supérieur et inférieur de la monture, et leur distance est parfaitement déterminée.

Afin de mettre en oeuvre la méthode selon l'invention, on fournit également une première installation 50 comprenant un dispositif d'enregistrement 52 représenté sur la Figure 2. Le dispositif d'enregistrement comporte au moins une caméra numérique 54 de type CCD orientée en direction du visage 12 du porteur à une distance D de celui-ci, équivalente à 2 m. Cette première installation comporte également des moyens de traitement non représentés. Ils incluent un module de traitement d'images permettant d'évaluer à partir des images fournies et enregistrées par la caméra 54, notamment la position relative des bords inférieur et supérieur de la monture et du reflet cornéen des pupilles 46, 48. Ils incluent également un module de calcul permettant d'évaluer l'inclinaison du plan moyen des logements 18, 20 par rapport à la verticale. Avantageusement, une lampe est installée au niveau de la caméra, de manière obtenir un reflet cornéen bien net.

Le porteur est équipé de la monture 10 laquelle est munie de son équipement calibré 26. La posture du porteur correspond ici à une vision de loin.

Dans cette position, on enregistre à l'aide de la caméra numérique 54 une image du visage 12 du porteur. On se reportera à présent sur la Figure 3 montrant les différents paramètres reproduits dans le plan de l'image PI1. Ainsi, l'axe optique A1 de la caméra numérique 54 coupe sensiblement le segment qui relie le reflet cornéen des deux pupilles 46, 48 et à équidistance de ces pupilles 46, 48. Aussi, l'axe optique A1 de la caméra numérique 54 est perpendiculaire au plan image PI1. On retrouve dans ce plan image, le bord supérieur A de la monture 10, la projection E du bord inférieur C, ainsi que la projection M du premier point d'intersection J de la première direction X1 du regard du porteur avec le plan moyen de logement PL défini par les logements 18, 20 de la monture 10. On observera que la première direction X1 du regard du porteur est confondue avec l'axe optique A1 de la caméra numérique 54.

On souhaite dans cette position connaître la distance entre le premier point d'intersection J et le bord inférieur C de la monture.

On détermine tout d'abord l'angle β. On connaît la distance TM qui sépare la caméra numérique 54 du point M projeté. Ce point M projeté est situé à équidistance des bords supérieur et inférieur du plan image PI1. On dénombre sur le plan image, d'une part les pixels qui séparent le point M et le point E et d'autre part les pixels qui séparent les deux points noirs 42, 44 de l'équipement calibré 26. Connaissant la distance réelle qui sépare ces deux points noirs 42, 44, on en déduit, en appliquant une règle de trois, la distance réelle ME. On en tire ainsi l'angle β du triangle AEC, car tg (π - β) =TM/ME.

Aussi, on en déduit l'angle pantoscopique α entre le plan image PI1 et le plan moyen de logement PL car dans le triangle AEC, α = π - β - arcsin [(AE sin β) / AC]. En tenant compte du calcul précédent, α = arctg[TM/ME] - arcsin [(AE sin β) / AC]. En outre, on détermine la longueur AE comme ci-dessus, en dénombrant les pixels entre le point M et le point E du plan image PI1 et en appliquant une règle de trois.

On détermine ainsi la position du point d'intersection J en calculant d'une part la distance AM comprise dans le plan image PI1 en dénombrant les pixels entre le point A et le point M et en appliquant une règle de trois et en divisant cette distance par cos α afin d'obtenir la distance AJ et d'autre part en soustrayant la distance AJ de la distance connue CA qui s'étend entre les deux bords supérieur et inférieur de la monture 10. On obtient ainsi la valeur de la distance CJ.

Par ailleurs, toujours dans cette posture en vision de loin telle que représentée sur la Figure 2 on mesure également la longueur qui s'étend entre les reflets cornéens des deux pupilles 46, 48, en dénombrant les pixels qui s'étendent entre les deux reflets projetés dans le plan image PI1 et en appliquant une règle de trois comme indiqué ci-dessus pour déterminer la distance réelle.

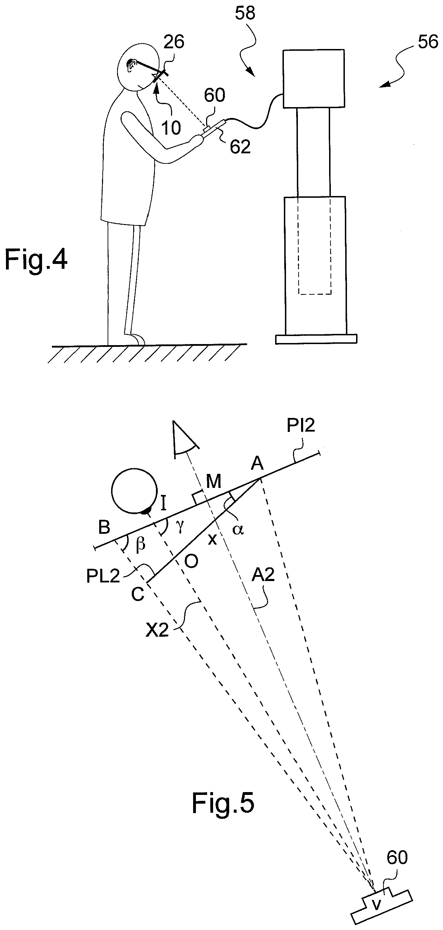

On va maintenant décrire en référence aux Figures 4 et 5 le mode de détermination de la position du point d'intersection de la direction du regard du porteur avec le plan moyen de logement dans une posture de vision de près.

On a représenté sur la Figure 4 le porteur équipé de la monture 10 laquelle est munie de son équipement calibré 26. Selon la méthode, on fournit en outre une seconde installation 56 comprenant un second dispositif d'enregistrement 58 comportan t une caméra numérique mobile 60 de type CCD. Cette caméra mobile 60 est montée à travers un support 62 sur lequel figurent des inscriptions. Aussi, il est demandé au porteur de la monture 10 de se mettre en position de lecture naturelle des inscriptions du support. Cette position relative du support et du visage 12 du porteur correspond à une posture de vision de près. L'axe optique de la caméra est alors orienté en direction du visage 12 du porteur à une distance calculable et selon une direction qui n'est pas nécessairement confondue avec la direction du regard du porteur.

Cette seconde installation 56 comporte également des moyens de traitement non représentés, et ils incluent un module de traitement d'images analogue au précédent permettant d'évaluer à partir des images fournies et enregistrées par la caméra numérique mobile 60, la distance de cette dernière par rapport à la monture 10, la position relative des bords inférieur et supérieur de la monture et du reflet cornéen des pupilles 46, 48. Ils incluent tout comme les précédents moyens de traitement, un module de calcul permettant d'évaluer l'inclinaison du plan moyen des logements 18, 20 par rapport à la verticale.

Dans cette posture de vision de près, on enregistre à l'aide de la caméra numérique mobile 60 une image du visage 12 du porteur. On se reportera à présent sur la Figure 5 montrant les différents paramètres reproduits dans le plan de l'image PI2.

L'axe optique A2 de la caméra numérique mobile 60 ne coupe plus le segment qui relie le reflet cornéen des deux pupilles 46, 48 mais il s'étend entre ce segment et un segment formé par les deux bords supérieurs A des logements de la monture.

En revanche, l'axe optique A2 de la caméra numérique mobile 60 est par nature toujours perpendiculaire au plan image PI2 qui sur la Figure 5 tangente le bord supérieur A des logements de la monture.

On retrouve ainsi dans ce plan image PI2, le bord supérieur A de la monture 10, la projection B du bord inférieur C, l'intersection M de l'axe optique A2 et du plan image PI2, ainsi que la projection I du second point d'intersection O de la seconde direction X2 du regard du porteur avec le second plan moyen de logement PL2 défini par les logements 18, 20 de la monture 10.

On souhaite dans cette position, comme dans la posture en vision de loin, connaître la distance entre le second point d'intersection O et le bord inférieur C de la monture.

Pour ce faire, on détermine tout d'abord la distance MV qui s'étend entre la caméra numérique mobile 60 et le point M. Cette distance est aisément déterminée car elle correspond à un nombre de pixels entre les deux points noirs 42, 44 de l'équipement calibré 26.

Ensuite, on détermine l'angle β. On connaît donc la distance MV qui sépare la caméra numérique mobile 60 du point M. On dénombre sur le plan image PI2, d'une part les pixels qui séparent le point M et le point B et d'autre part les pixels qui séparent les deux points noirs 42, 44 de l'équipement calibré 26. On en déduit, en appliquant une règle de trois, la distance réelle MB. On en tire ainsi l'angle β du triangle ABC, car tg β =MV/MB.

Aussi, on en déduit l'angle α entre le plan image PI2 et le plan moyen de logement PL2 car dans le triangle ABC, α = π - β - arcsin [(AB sin β) / AC]. En outre, on détermine la longueur AI comme ci-dessus, en dénombrant les pixels entre le point M et le point I du plan image PI2 et en appliquant une règle de trois. De plus, on calcule l'angle γ = arctg [MV/MI] de manière à obtenir la distance AO = AI [sin β/ sin (α + γ)].

On détermine ainsi la distance recherchée CO en soustrayant la distance AO de la distance connue AC qui s'étend entre les deux bords supérieur et inférieur de la monture 10.

On observera que l'angle α entre le plan image PI2 et le plan moyen de logement PL2, peut être obtenu de manière connue au moyen de l'équipement calibré 26, par triangulation, grâce à un troisième repère situé au centre du pont 28 et qui s'étend en saillie du pont.

Par ailleurs, toujours dans cette posture en vision de près telle que représentée sur la Figure 5 on mesure également la longueur qui s'étend entre les reflets cornéens des deux pupilles 46, 48, en dénombrant les pixels qui s'étendent entre les deux reflets projetés dans le plan image PI2 et en appliquant une règle de trois comme indiqué ci-dessus pour déterminer la distance réelle.

Ensuite, on évalue la distance qui s'étend entre les points d'intersection J pour la posture en vision de loin et O pour la posture en vision de près, en soustrayant la valeur CO obtenue par la posture en vision de près de la valeur CJ obtenu par la posture en vision de loin.

Enfin, on peut alors sélectionner des lentilles ophtalmiques progressives dont la longueur de progression correspond à ladite distance évaluée entre les points d'intersection O et J.

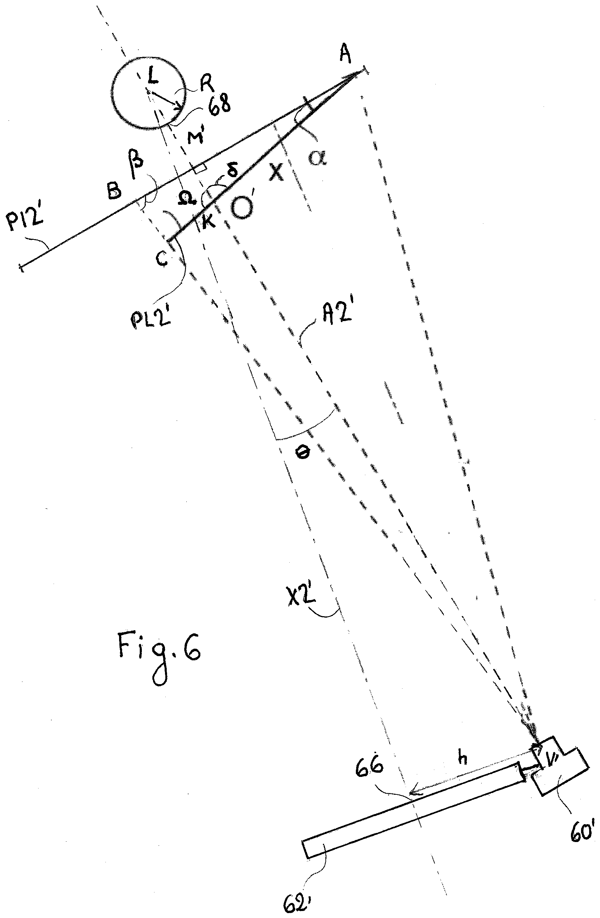

Selon le principal mode de mise en oeuvre de l'invention illustré sur la Figure 6, où les éléments et points strictement analogues au précédent illustré sur la Figure 5 présentent les mêmes références, tandis que les éléments analogues jouant le même rôle sont affectés d'une même référence affectée d'un signe prime « ' », le support est constitué d'un écran d'affichage portable 62', tandis que la caméra numérique CCD 60' est installée de manière solidaire sur ledit écran d'affichage portable 62'. Ainsi qu'on va l'expliquer ci-après, l'écran d'affichage portable 62' équipé, présente un double avantage. Il permet à la fois d'orienter l'axe optique de la caméra 60' vers le centre de l'oeil L, ce qui permet d'améliorer la précision des mesures, et également de constituer le support de lecture en vision de près pour le porteur. Toutefois une correction est nécessaire afin de tenir compte de la position de la caméra 60' par rapport à l'écran d'affichage portable 62', et en particulier lorsque l'axe optique de la caméra est incliné par rapport à l'écran d'affichage 62'.

Ainsi, par rapport à la Figure 5, la position relative du support 62 et maintenant de l'écran d'affichage 62' est rigoureusement identique. En revanche, la caméra numérique 60' n'est plus située dans l'axe optique de l'oeil, mais l'axe optique de la caméra 60', lui, vient couper l'oeil sensiblement en son centre L. En outre, la seconde direction du regard X2' est orientée vers une médiane horizontale 66 de l'écran d'affichage 62', qu'il vient couper sensiblement perpendiculairement. Cette médiane horizontale 66 est ici perpendiculaire au plan de la figure.

Avant de décrire les conséquences géométriques de cet agencement et d'en déterminer les principaux paramètres utiles, on expliquera les conditions opératoires qui permettent d'y parvenir.

Tout d'abord, comme pour le support 62 du précédent mode de mise en oeuvre, l'écran d'affichage portable 62', est saisi de chaque côté par le porteur, qui le porte à sa distance de lecture de près. Cette distance entre le centre de l'oeil L et l'écran est voisine de 40 cm. Aussi, l'orientation de la caméra 60' par rapport à l'écran d'affichage portable 62' est préréglée de façon que l'axe optique A2' de la caméra 60' vienne croiser la normale à l'écran 62' coupant le centre de la médiane précitée, et ici confondue avec la seconde direction du regard X2' à environ 40 cm de l'écran 62'.

Ensuite, l'image obtenue grâce à la caméra 60', correspondant au plan image PI2' est retransmise sur l'écran d'affichage portable 62' en temps réel. En outre, on affiche sur l'écran d'affichage portable 62' la ligne médiane horizontale 66, qui sépare l'écran en deux parties égales haute et basse.

Ainsi, on demande au porteur chaussé de la monture munie de l'équipement calibré 26, non seulement d'ajuster l'écran d'affichage portable 62' dans sa position normale de lecture de près, mais aussi de l'orienter de façon que ses yeux soient centrés latéralement et que la ligne médiane vienne les couper au niveau du reflet cornéen 68. Une fois dans cette position, on enregistre l'image. En effet, dans cette position l'axe optique A2' de la caméra 60' vient couper le centre de l'oeil L. La Figure 6 illustre ainsi schématiquement de côté la situation.

On observera que l'axe optique A2' coupe le plan moyen de logement PL2' en un second point O', tandis que le second point d'intersection de la seconde direction du regard X2' vient couper le plan moyen de logement PL2' en un point K.

C'est bien évidemment ici la valeur de la distance CK qu'il convient de rechercher.

On détermine tout d'abord aisément la valeur de la distance AO' car l'angle α entre le plan image PI2' et le plan moyen de logement PL2' dans le triangle ABC est déjà connu et la distance AM' dans le plan image PI2' est déterminée par le calcul du nombre de pixels et l'application d'une règle de trois. L'axe optique A2' venant couper par définition le plan image PI2' de façon perpendiculaire, la distance AO' est donc équivalente à AM/cos a.

Il convient à présent de déterminer la distance qui sépare le point O' du point K.

Tout d'abord, on connaît la distance qui sépare le point V' de la caméra 60' au point O' du plan de logement PL2' en évaluant le nombre de pixels sur l'équipement calibré 26 et en opérant une règle de trois. De plus, on a déjà calculé la distance qui sépare selon cette direction de l'axe optique A2', le plan de logement PL2' de la cornée de l'oeil au niveau du reflet cornéen 68 et elle vaut environ 1.3 cm. Et on connaît le rayon moyen R d'un oeil, environ 0,8 cm, soit également la distance totale LO'. Aussi, on en déduit tout naturellement la distance du centre de l'oeil L au centre optique V' de la caméra 60'. Par ailleurs on connaît la distance h qui sépare la caméra 60' de la ligne médiane horizontale 66. Par conséquent, le centre de l'oeil L, le centre optique de la caméra 60' et le point d'intersection de la seconde direction du regard X2' avec la ligne médiane 66 formant un triangle rectangle, la valeur de l'angle θ entre la seconde direction du regard X2' et l'axe optique A2', et plus précisément sin θ, est connu et vaut h/LV'.

Maintenant, au niveau du triangle L, O', K, on reconnaît la distance LO', et le sin θ de l'angle entre les segments LK et LO'. Il nous faut alors déterminer l'angle Ω entre les segments LO' et O'K, pour pouvoir évaluer le segment O'K.

L'angle Ω, est équivalent à la différence entre π et l'angle δ entre le segment O'A et le segment O'M'. Or, l'angle δ vaut π/2- α, et par conséquent l'angle Ω vaut α + π/2.

Aussi, connaissant la longueur d'un côté d'un triangle commun à deux de ses angles, on en déduit la longueur d'un autre côté. Et en l'espèce, O'K est égale au rapport du produit O'L.sin θ et de sin (θ + α + π/2). Toutes ces valeurs étant connues, on en déduit tout naturellement la valeur O'K, et partant la valeur du segment AK, et de façon plus intéressante la valeur de CK, entre le bord inférieur B de la monture et le centre de la vision de près K sur la monture.

On observera que le calcul de la distance O'K est sensiblement différent lorsque la caméra 62' est orientée de manière à ce que son axe optique A2' soit perpendiculaire à l'écran d'affichage portable 62', car en ce cas, cette dernière est parallèle au plan image PI2'. Aussi ce n'est plus le sinus de l'angle θ qui est déterminé mais sa tangente h/V'L.

## Revendications

1. Méthode de sélection de lentilles ophtalmiques progressives pour une monture et un porteur donnés, les lentilles ophtalmiques progressives présentant une zone de vision de loin et une zone de vision de près espacées l'une de l'autre d'une longueur de progression, ladite monture donnée présentant deux logements aptes à recevoir respectivement une lentille Ophtalmique progressive, lesdits deux logements définissant un plan moyen de logement, ladite méthode étant du type comprenant les étapes suivantes :
a) on chausse ledit porteur donné avec ladite monture donnée ;
b) on détermine par rapport à ladite monture, la position d'un premier point d'intersection d'une première direction du regard dudit porteur dans une posture de vision de loin avec ledit plan moyen de logement ;
c) on détermine par rapport à ladite monture, la position d'un second point d'intersection d'une seconde direction du regard dudit porteur dans une posture de vision de près avec ledit plan moyen de logement ;
d) on évalue la distance qui s'étend entre lesdits points d'intersection ; et,
e) on sélectionne des lentilles ophtalmiques progressives dont la longueur de progression correspond à ladite distance évaluée entre lesdits points d'intersection ;
**caractérisée en ce qu'**on fournit en outre un écran d'affichage portable (62') et une caméra (60') installée sur ledit écran d'affichage portable (62'), ladite caméra fournissant une image retransmise sur ledit écran d'affichage portable, et **en ce qu'**à l'étape c), ledit porteur ajuste ledit écran d'affichage portable dans ladite posture de vision de près, et oriente l'axe optique de ladite caméra (60'), vers le centre de l'oeil L et **en ce qu'**on affiche sur l'écran d'affichage portable la ligne médiane horizontale (66), qui sépare l'écran en deux parties égales haute et basse et **en ce que** ledit porteur oriente ledit écran d'affichage portable de façon que ses yeux soient centrés latéralement et que la ligne médiane horizontale (66) vienne couper ses yeux au niveau du reflet cornéen (68).

2. Méthode de sélection selon la revendication 1, **caractérisée en ce qu'**on équipe ladite monture d'un équipement calibré présentant trois points de repères espacés respectivement les uns des autres d'une distance déterminée.

3. Méthode de sélection selon la revendication 2, **caractérisée en ce qu'**à l'étape b), on enregistre selon une direction voisine de ladite première direction du regard, une première image de ladite monture équipée dudit équipement calibré et des pupilles des yeux dudit porteur pour déterminer la position dudit premier point d'intersection par rapport à ladite monture.

4. Méthode de sélection selon la revendication 3, **caractérisée en ce qu'**on fournit une première caméra et **en ce qu'**on ajuste ladite première caméra à distance dudit porteur chaussant ladite monture équipée dudit équipement calibré et selon une direction voisine de ladite première direction du regard pour enregistrer ladite première image.

5. Méthode de sélection selon l'une quelconque des revendications 2 à 4, **caractérisée en ce qu'**à l'étape c), on enregistre selon une direction voisine de ladite seconde direction du regard, une seconde image de ladite monture équipée dudit équipement calibré et des pupilles des yeux dudit porteur pour déterminer la position dudit second point d'intersection par rapport à ladite monture.

6. Ensemble de traitement automatique permettant de sélectionner des lentilles ophtalmiques progressives pour une monture donnée chaussée sur porteur donné, les lentilles ophtalmiques progressives présentant une zone de vision de loin et une zone de vision de près espacées l'une de l'autre d'une longueur de progression, ladite monture donnée présentant deux logements aptes à recevoir respectivement une lentille ophtalmique progressive, lesdits deux logements définissant un plan moyen de logement, ledit ensemble comprenant :
- des premiers moyens de détermination pour déterminer par rapport à ladite monture, la position d'un premier point d'intersection d'une première direction du regard dudit porteur dans une posture de vision de loin avec ledit plan moyen de logement ;
- des seconds moyens de détermination pour déterminer par rapport à ladite monture, la position d'un second point d'intersection d'une seconde direction du regard dudit porteur dans une posture de vision de près avec ledit plan moyen de logement ;
- des moyens d'évaluation pour évaluer la distance qui s'étend entre lesdits points d'intersection ; et,
- des moyens de sélection pour sélectionner des lentilles ophtalmiques progressives dont la longueur de progression correspond à ladite distance évaluée entre lesdits points d'intersection ;
**caractérisé en ce que** lesdits seconds moyens de détermination comprennent un écran d'affichage portable et une caméra installée sur ledit écran d'affichage portable, ladite caméra fournissant une image retransmise sur ledit écran d'affichage portable (62'), et **en ce qu'**on affiche sur l'écran d'affichage portable la ligne médiane horizontale (66), qui sépare l'écran en deux parties égales haute et basse afin de permettre au dit porteur d'orienter le dit écran d'affichage portable de façon que ses yeux soient centrés latéralement et que la ligne médiane horizontale (66) vienne couper ses yeux au niveau du reflet cornéen (68).

7. Ensemble de traitement automatique selon la revendication 6, **caractérisé en ce que** lesdits premiers et seconds moyens de détermination comprennent un équipement calibré destiné à équiper ladite monture, et présentant trois points de repères espacés respectivement les uns des autres d'une distance déterminée.

8. Ensemble de traitement automatique selon la revendication 6 ou 7, **caractérisé en ce que** lesdits seconds moyens de détermination comprennent en outre des moyens d'enregistrement et de calcul, pour enregistrer les images fournies par ladite caméra et pour calculer ladite position du second point d'intersection de la seconde direction du regard dudit porteur dans une posture de vision de près avec ledit plan moyen de logement.

## Patentansprüche

1. Verfahren zur Auswahl von Gleitsichtgläsern für ein gegebenes Gestell und einen gegebenen Träger, wobei die Gleitsichtgläser eine Fernsichtzone und eine Nahsichtzone aufweisen, die voneinander um eine Progressionslänge beabstandet angeordnet sind, wobei das gegebene Gestell zwei Fassungen aufweist, die geeignet sind, jeweils ein Gleitsichtglas aufzunehmen, wobei die zwei Fassungen eine mittlere Fassungsebene definieren, wobei das Verfahren vom Typ ist, das die folgenden Schritte aufweist:
a) dem gegebenen Träger wird das gegebene Gestell aufgesetzt;
b) man bestimmt bezüglich des Gestells die Position eines ersten Schnittpunktes einer ersten Blickrichtung des Trägers in Fernsichthaltung mit der mittleren Ebene der Fassung;
c) man bestimmt bezüglich des Gestells die Position eines zweiten Schnittpunktes einer zweiten Blickrichtung des Trägers in Nahsichthaltung mit der mittleren Ebene der Fassung;
d) man evaluiert den Abstand, der sich zwischen diesen Schnittpunkten erstreckt; und
e) man wählt die Gleitsichtgläser aus, bei denen die Progressionslänge dem evaluierten Abstand zwischen den Schnittpunkten entspricht;
**dadurch gekennzeichnet, dass** man außerdem einen tragbaren Anzeigebildschirm (62 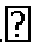 ) und eine auf dem tragbaren Anzeigebildschirm (62 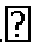 ) installierte Kamera (60 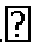 ) bereitstellt, wobei die Kamera ein Bild liefert, das auf den tragbaren Anzeigebildschirm übertragen wird,
und dadurch, dass bei Schritt c) der Träger den tragbaren Anzeigebildschirm in der Nahsichthaltung einstellt und die optische Achse der Kamera (60 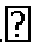 ) hin zum Augenmittelpunkt L ausrichtet, und dadurch, dass auf dem tragbaren Anzeigebildschirm die horizontale Mittellinie (66) angezeigt wird, die den Bildschirm in zwei gleiche Teile aufteilt, und zwar einen oberen und einen unteren Teil, und dadurch, dass der Träger den tragbaren Anzeigebildschirm derart ausrichtet, dass seine Augen in seitlicher Richtung zentriert sind und dass die horizontale Mittellinie (66) seine Augen auf Höhe des Cornea-Reflexes (68) schneidet.

2. Verfahren zur Auswahl nach Anspruch 1, **dadurch gekennzeichnet, dass** man das Gestell mit einer kalibrierten Apparatur ausrüstet, die drei Markierungspunkte aufweist, welche voneinander um einen bestimmten Abstand entfernt angeordnet sind.

3. Verfahren zur Auswahl nach Anspruch 2, **dadurch gekennzeichnet, dass** man bei Schritt b) gemäß einer Richtung nahe der ersten Blickrichtung ein erstes Bild des mit der kalibrierten Apparatur ausgerüsteten Gestells und der Pupillen der Augen des Trägers aufnimmt, um die Position des ersten Schnittpunktes bezüglich des Gestells zu bestimmen.

4. Verfahren zur Auswahl nach Anspruch 3, **dadurch gekennzeichnet, dass** man eine erste Kamera bereitstellt, und dadurch, dass man die erste Kamera auf eine Entfernung zum Träger, der das mit der kalibrierten Apparatur ausgerüstete Gestell trägt, und entlang einer Richtung nahe der ersten Blickrichtung einstellt, um das erste Bild aufzuzeichnen.

5. Verfahren zur Auswahl nach einem der Ansprüche 2 bis 4, **dadurch gekennzeichnet, dass** man bei Schritt c) entlang einer Richtung nahe der zweiten Blickrichtung ein zweites Bild des mit der kalibrierten Apparatur ausgerüsteten Gestells und der Pupillen der Augen des Trägers aufnimmt, um die Position des zweiten Schnittpunktes bezüglich des Gestells zu bestimmen.

6. Anordnung zur automatischen Behandlung, die ein Auswählen von Gleitsichtgläsern für ein gegebenes Gestell ermöglicht, das einem gegebenen Träger aufgesetzt wurde, wobei die Gleitsichtgläser eine Fernsichtzone und eine Nahsichtzone aufweisen, die voneinander um eine Progressionslänge beabstandet angeordnet sind, wobei das gegebene Gestell zwei Fassungen aufweist, die geeignet sind, jeweils ein Gleitsichtglas aufzunehmen, wobei die zwei Fassungen eine mittlere Fassungsebene definieren, wobei die Anordnung aufweist:
erste Bestimmungseinrichtungen, um bezüglich des Gestells die Position eines ersten Schnittpunktes einer ersten Blickrichtung des Trägers in Fernsichthaltung mit der mittleren Ebene der Fassung zu bestimmen;
zweite Bestimmungseinrichtungen, um bezüglich des Gestells die Position eines zweiten Schnittpunktes einer zweiten Blickrichtung des Trägers in Nahsichthaltung mit der mittleren Ebene der Fassung zu bestimmen;
Evaluierungseinrichtungen, um den Abstand zu evaluieren, der zwischen den Schnittpunkten vorhanden ist; und
Auswahleinrichtungen, um die Gleitsichtgläser auszuwählen, bei denen die Progressionslänge dem evaluierten Abstand zwischen den Schnittpunkten entspricht;
**dadurch gekennzeichnet, dass** die zweiten Bestimmungseinrichtungen einen tragbaren Anzeigebildschirm und eine auf dem tragbaren Anzeigebildschirm installierte Kamera beinhalten, wobei die Kamera ein Bild liefert, das auf den tragbaren Anzeigebildschirm (62 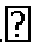 ) übertragen wird, und dadurch, dass auf dem tragbaren Anzeigebildschirm die horizontale Mittellinie (66) angezeigt wird, die den Bildschirm in zwei gleiche Teile aufteilt, und zwar einen oberen und einen unteren Teil, um dem Träger zu ermöglichen, den tragbaren Anzeigebildschirm derart auszurichten, dass seine Augen in seitlicher Richtung zentriert sind und dass die horizontale Mittellinie (66) seine Augen auf Höhe des Cornea-Reflexes (68) schneidet.

7. Anordnung zur automatischen Behandlung nach Anspruch 6, **dadurch gekennzeichnet, dass** die ersten und zweiten Bestimmungseinrichtungen eine kalibrierte Apparatur beinhalten, mit der das Gestell ausgerüstet werden soll, und drei Markierungspunkte aufweisen, die voneinander um einen bestimmten Abstand entfernt angeordnet sind.

8. Anordnung zur automatischen Behandlung nach Anspruch 6 oder 7, **dadurch gekennzeichnet, dass** die zweiten Bestimmungseinrichtungen außerdem Aufzeichnungs- und Berechnungseinrichtungen aufweisen, um die von der Kamera gelieferten Bilder aufzuzeichnen und die Position des zweiten Schnittpunkts der zweiten Blickrichtung des Trägers in Nahsichthaltung mit der mittleren Ebene der Fassung zu berechnen.

## Claims

1. Method of selecting progressive ophthalmic lenses for a given frame and wearer, the progressive ophthalmic lenses having a far vision zone and a near vision zone separated from each other by a progression length, said given frame having two recesses into each of which a progressive ophthalmic lens fits, said two recesses defining a median recess plane, said method being of the type that comprises the following steps:
a) said wearer is fitted with said given frame;
b) the position of a first intersection point of a first view direction of said wearer in a far vision posture and said median recess plane, is determined relative to said frame;
c) the position of a second intersection point of a second view direction of said wearer in a near vision posture and said median recess plane, is determined relative to said frame;
d) the distance between said intersection points is evaluated, and,
e) progressive ophthalmic lenses for which the progression length corresponds to said distance evaluated between said intersection points are selected
**characterised in that** a portable display screen (62') and a camera (60') installed on said portable display screen (62') are also provided, said camera supplying an image displayed on said portable display screen,
and **in that** in step c), said wearer adjusts said portable display screen in said near vision posture, and orients the optical axis of said camera (60') towards the centre of the eye L and **in that** the horizontal median line (66) that separates the screen into two equal top and bottom parts is displayed on the portable display screen, and **in that** said wearer orients said portable display screen such that his or her eyes are centred laterally and **in that** the horizontal median line (66) intersects his or her eyes at the corneal reflex (68)

2. Selection method according to claim 1, **characterised in that** said frame is fitted with calibrated equipment with three identification points at a determined separation distance from each other.

3. Selection method according to claim 2, **characterised in that** in step b), a first image of said frame fitted with said calibrated equipment and the pupils of the eyes of said wearer along a direction close to said first view direction is recorded, to determine the position of said first intersection point relative to said frame.

4. Selection method according to claim 3, **characterised in that** a first camera is provided and **in that** said first camera is adjusted at a distance from said wearer of said frame fitted with said calibrated equipment and along a direction close to said first view direction to record said first image.

5. Selection method according to any one of claims 2 to 4, **characterised in that** in step c), a second image of said frame fitted with said calibrated equipment and the pupils of the eyes of said wearer along a direction close to said second view direction is recorded, to determine the position of said second intersection point relative to said frame.

6. Automatic processing assembly for the selection of progressive ophthalmic lenses for a given frame worn by a given wearer, the progressive ophthalmic lenses having a far vision zone and a near vision zone separated from each other by a progression length, said given frame having two recesses into each of which a progressive ophthalmic lens fits, said two recesses defining a median recess plane, said assembly comprising:
- first determination means to determine the position of a first intersection point of a first view direction of said wearer in a far vision posture and said median recess plane, relative to said frame;
- second determination means to determine the position of a second intersection point of a second view direction of said wearer in a near vision posture and said median recess plane, relative to said frame;
- evaluation means to evaluate the distance between said intersection points; and,
- selection means to select progressive ophthalmic lenses for which the progression length corresponds to said distance evaluated between said intersection points;
**characterised in that** said second determination means comprise a portable display screen and a camera installed on said portable display screen, said camera supplying an image displayed on said portable display screen (62'),and **in that** the horizontal median line (66) that separates the screen into two equal top and bottom parts is displayed on the display screen, such that said wearer can orient said portable display screen such that his or her eyes are centred laterally and **in that** the horizontal median line (66) intersects his or her eyes at the corneal reflex (68).

7. Automatic processing assembly according to claim 6, **characterised in that** said first and second determination means comprise calibrated equipment that will be fitted on said frame, and having three identification points located at a determined distance from each other.

8. Automatic processing assembly according to claim 6 or 7, **characterised in that** said second determination means also comprise recording and calculation equipment, to save images taken by said camera and to calculate said position of the second intersection point of the second view direction of said wearer in a near viewing posture with said median recess plane.
